# EUROPEAN PATENT SPECIFICATION

(11) **EP 3 297 546 B1**
(45) Date of publication and mention of the grant of the patent: **22.10.2025**
(21) Application number: 16727071.9
(22) Date of filing: 20.05.2016
(51) Int. Cl.: A61B 17/12, A61M 25/00, A61M 25/10

(54) **DEVICE FOR CLOSING OFF A VEIN**
VORRICHTUNG ZUM VERSCHLUSS EINER VENE
DISPOSITIF D'OCCLUSION D'UNE VEINE

(30) Priority: 21.05.2015 US 201514718865; 22.12.2015 US 201514978021
(43) Date of publication of application: 28.03.2018
(73) Proprietor: BASIS Medical, LLC, Atlanta, GA 30307 (US)
(72) Inventor: Martin, David A., Atlanta, GA 30307 (US)
(74) Representative: Appleyard Lees IP LLP
(86) International application number: PCT/US2016/033510
(87) International publication number: WO 2016/187530

(56) References cited:
- EP-A2- 0 970 718
- WO-A1-95/11719
- US-A- 5 224 938
- US-A- 5 662 609
- US-A1- 2009 318 855
- US-A1- 2014 163 525

## Description

### FIELD OF THE INVENTION

The following invention generally relates to the field of body vessel seclusion.

### BACKGROUND OF THE INVENTION

For certain medical conditions, it may be necessary or desirable to seclude (i.e., to close off, collapse, or significantly narrow) a body vessel such as a vein or artery. One situation in which seclusion may be desirable is in the treatment of varicose veins, which are swollen, twisted, or enlarged veins that may be visible under a patient's skin. By closing off the varicose vein, blood ceases to flow in the varicose vein and is naturally redirected to healthy veins. Over time, the closed-off vein may be completely absorbed into surrounding tissue.

There are several techniques currently in use for secluding a blood vessel such as a varicose vein. Examples of these techniques include surgery, heat ablation, and chemical treatment. Surgically, veins may be subjected to a seclusion procedure known as ligation. During ligation, a small incision may be made near the target vein and the vein may be tied off. The ligated vein may be left in place and absorbed into surrounding tissue, as noted above. Alternatively, the ligated vein may be removed by a process known as "stripping" the vein. The surgical treatment of veins in this manner is sometimes referred to as phlebectomy.

The surgical treatment of varicose veins is generally effective, but may carry certain risks and disadvantages. The procedure is relatively invasive compared to other varicose vein treatment methods, and accordingly may be painful for some patients. Surgical treatment of varicose veins also carries a risk of nerve injury, may require the use of general anesthesia and an overnight hospital stay, and may require a relatively long recovery time. Accordingly, other types of vein treatment have been developed. These treatments generally involve damaging the walls of the vein, which causes the vein walls to collapse, close, or narrow. For example, in heat ablation treatment, a heat source (typically a laser or radio frequency transmitter) may be inserted into the vein through a catheter. Upon reaching a target area of the vein, the heat source may be turned on for a predetermined period of time, which damages the target area of the vein and causes scar tissue to form on the inner walls of the vein. The build-up of scar tissue closes the vein. Problematically, the same heat that damages the vein can also damage surrounding tissue and nearby nerves. It can also cause skin burns and blood clots, and may not be appropriate for all types of veins.

The vein walls can also be damaged chemically in a procedure known as sclerotherapy. In sclerotherapy, a chemical known as a sclerosing agent may be injected into the vein. The sclerosing agent may damage the walls of the vein and cause the vein to narrow or close. However, in order to be effective, the sclerosing agent needs to remain in contact with the inside walls of the target area of the vein for some time (e.g., up to one minute). This is difficult to achieve using conventional sclerotherapy procedures because the sclerosing agent may be quickly washed away by the flow of blood through the vein. As a result, the sclerosing agent may be diluted and flow to other portions of the body, and hence the sclerosing agent may not be sufficiently effective to close the vein upon an initial application. Accordingly, patients may need several treatment sessions with one or more injections of sclerosing agent applied in each session. In order to address these issues, a new sclerotherapy treatment method called catheter-directed foam sclerotherapy ("CDFS") has recently been employed. In this method, a catheter is inserted into the vein and moved to the target site. The sclerosing agent is injected into the vein through the catheter in the form of a foam. Because the agent is a foam, it is relatively more difficult for the blood flow to dilute and remove the sclerosing agent. Therefore, as compared to conventional sclerotherapy, CDFS allows the sclerosing agent to be present at the target site for a relatively longer period of time, in a relatively larger concentration. Nonetheless, the sclerosing agent will still be washed away from the target site due to the flow of the blood in the vein, so repeated treatments may remain necessary.

Therefore there at least remains a need in the art for a method and device for secluding a body vessel such that a sclerosing agent may be maintained at the target site without being washed away due to blood flow in the vessel.

US5662609A describes a technique for treating diseased portions of tissue lumens by introducing at least one therapeutic agent at the diseased region.

### SUMMARY OF THE INVENTION

One or more embodiments of the invention may address one or more of the aforementioned problems. In one aspect, a device for secluding a body vessel is provided according to claim 1.

### BRIEF DESCRIPTION OF THE DRAWINGS

Exemplary embodiments now will be described more fully hereinafter with reference to the accompanying drawings, in which some, but not all embodiments of the invention are shown. The present invention may be embodied in many different forms and should not be construed as limited to the embodiments set forth herein; rather, these embodiments are provided so that this disclosure will satisfy applicable legal requirements and demonstrate exemplary embodiments of the invention. Repeat use of reference characters in the present specification and drawings is intended to represent same or analogous features or elements of the invention.
FIG. 1 illustrates a partial view of a device for secluding a body vessel in a pre-deployed form;
FIG. 2 illustrates a device for secluding a body vessel including the lumens;
FIG. 3 illustrates a cross-section of a lumen assembly in a device for secluding a body vessel;
FIG. 4 illustrates a partial view of a device for secluding a body vessel in a deployed form;
FIG. 5 illustrates a body vessel with an identified area to be secluded;
FIG. 6 illustrates a block diagram of a method of secluding a body vessel; and
FIG. 7 illustrates a block diagram of a method of secluding a body vessel.

### DETAILED DESCRIPTION OF EXEMPLARY EMBODIMENTS

Reference will now be made in detail to exemplary embodiments of the invention, one or more examples of which are illustrated in the accompanying drawings. Each example is provided by way of explanation of the invention, not limitation of the invention. In fact, it will be apparent to those skilled in the art that modifications and variations can be made in the present invention without departing from the scope thereof, as long as the modifactions and variations fall into the scope of the invention defined in appended claim 1. For instance, features illustrated or described as part of one embodiment may be used on another embodiment to yield a still further embodiment.

In contrast to conventional treatment methods and devices, the exemplary embodiments disclosed herein may be less invasive and may require less recovery time. Moreover, the exemplary embodiments disclosed herein may eliminate the need for a general anesthetic, instead relying on a local anesthetic. In this regard, the exemplary embodiments disclosed herein may reduce procedural risks and further decrease required recovery time. In addition, the exemplary embodiments disclosed herein may be associated with a reduced risk of nerve damage, skin damage, and recovery pain.

As used herein, the term "body vessel" may comprise any lumen or other similar region in a body, such as a blood vessel or the intestines. Although specific examples are provided herein with reference to veins, one of ordinary skill in the art will recognize that the device and methods disclosed herein are not limited to these particular examples but rather may be employed in any suitable body vessel.

The term "seclusion", as used herein, may refer to the narrowing, collapsing, or closing off of a body vessel. Accordingly, seclusion may be distinct from therapies intended to open or widen a vessel and from therapies intended to prevent the vessel from narrowing. The term "two-point seclusion", as used herein, may refer to secluding the body vessel at two points with a narrowed, collapsed, or closed space between the points.

For ease of reference, exemplary embodiments will be described in terms of use in human subjects. It will be understood, however, that such descriptions are not limited to use to humans, but will also include use in other animals unless explicitly stated otherwise. Moreover, although a catheter is referred to herein, one of ordinary skill in the art will recognize that a catheter is merely an exemplary device as disclosed herein.

According to the invention, a device for closing off a vein is provided. The device for closing off a vein includes a distal balloon, a proximal balloon, an aspiration port positioned adjacent to the distal balloon, an injection port positioned adjacent to the proximal balloon, and a lumen assembly. The lumen assembly comprises a distal balloon lumen operably coupled to the distal balloon, a proximal balloon lumen operably coupled to the proximal balloon, an aspiration port lumen operably coupled to the aspiration port, and an injection port lumen operably coupled to the injection port. The distal balloon and the proximal balloon define a treatment chamber therebetween, and the aspiration port and the injection port are positioned within the treatment chamber on the lumen assembly.

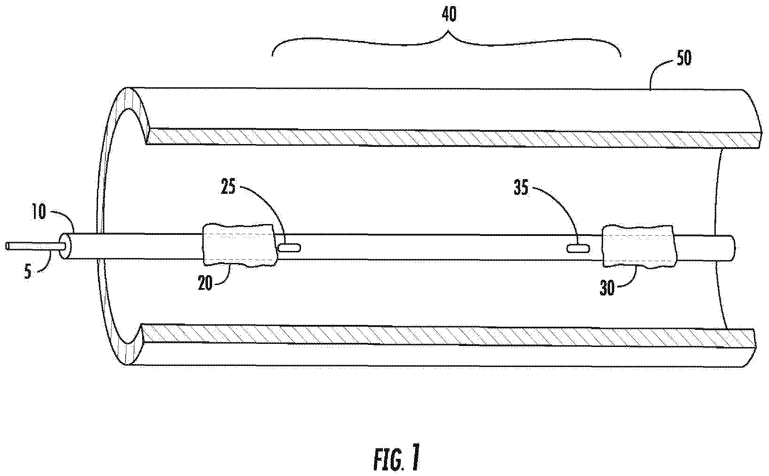

FIG. 1, for instance, illustrates a partial view of a device for closing off a vein. in a pre-deployed form according to the invention. As shown in FIG. 1, for example, the device may be a catheter. The device includes a lumen assembly 10, a proximal balloon 20, an injection port 25 positioned adjacent to the proximal balloon 20, a distal balloon 30, and an aspiration port 35 positioned adjacent to the distal balloon 30. The proximal balloon 20 and the distal balloon 30 define a treatment chamber 40 therebetween inside of a body vessel 50 when the balloons 20, 30 are inflated. In this regard, for example, a chemical agent may be introduced into the treatment chamber 40 to seclude the body vessel 50 within the treatment chamber 40. The balloons 20, 30 may be made of any suitable material as understood by one of ordinary skill in the art including, but not limited, to polymeric materials. In accordance with certain embodiments, for example, the body vessel 50 may comprise at least one of a varicose vein, a portal vein, a perforator vein, a superficial vein, a peripheral vein, an arteriovenous malformation, or any combination thereof. The catheter may be of any length suitable for secluding a variety of body vessels as understood by one of ordinary skill in the art (e.g., 100 cm).

According to certain embodiments, for instance, the treatment chamber 40 may comprise a length from about 3 cm to about 15 cm. In some embodiments, for example, the treatment chamber 40 may comprise a length from about 5 cm to about 10 cm. In further embodiments, for instance, the treatment chamber 40 may comprise a length from about 6 cm to about 8 cm. In certain embodiments, for example, the treatment chamber 40 may comprise a length of about 7 cm. As such, in certain embodiments, the treatment chamber 40 may comprise a length from at least about any of the following: 3, 4, 5, 6, and 7 cm and/or at most about 15, 12, 10, 9, 8, and 7 cm (e.g., about 4-9 cm, about 6-12 cm, etc.).

The lumen assembly 10 may comprise a flexible tube having several hollow lumens therein as described in more detail below. The individual lumens may not be very flexible. For example, the individual lumens within the lumen assembly 10 may only bend and/or move from about 2 mm to about 3 mm. However, the lumen assembly 10 may be sufficiently flexible to navigate through the body vessels of an individual. For instance, the lumen assembly 10 may be used to guide the device into position inside the body vessel 50, for example, via a guide wire 5.

According to certain embodiments, for instance, the guide wire 5 may comprise a diameter from about .001 cm to about .025 cm. In some embodiments, for example, the guide wire 5 may comprise a diameter from about .01 cm to about .02 cm. In further embodiments, for instance, the guide wire 5 may comprise a diameter from about .015 cm to about .019 cm. In other embodiments, for example, the guide wire 5 may comprise a diameter of about .018 cm. As such, in certain embodiments, the guide wire 5 may comprise a diameter from at least about any of the following: .001, .005, .01, .015, .016, .017, and .018 cm and/or at most about .025, .024, .023, .022, .021, .02, .019, and .018 cm (e.g., about .01-.019 cm, about .017-.024 cm, etc.). However, the guide wire 5 may comprise any guide wire suitable for use with the device disclosed herein as understood by one of ordinary skill in the art.

In accordance with the invention, the lumen assembly 10 includes the injection port 25 and the aspiration port 35 to introduce and evacuate fluids respectively. The injection port 25 and the aspiration port 35 are positioned on the lumen assembly 10 within the treatment chamber 40 created by the inflated proximal balloon 20 and the distal balloon 30. In some embodiments, for example, each of the aspiration port 35 and the injection port 25 comprise a port orifice and a one-way valve at the port orifice. In this regard, for instance, the aspiration port 35 may evacuate blood and other bodily fluids from the treatment chamber 40 to provide an empty area for the chemical agent to occupy and to prevent the chemical agent from being diluted. Additionally, the aspiration port 35 may evacuate the chemical agent from the treatment chamber 40 after treatment. Moreover, the injection port 25 may introduce the chemical agent into the treatment chamber 40 to initiate seclusion of the body vessel 50.

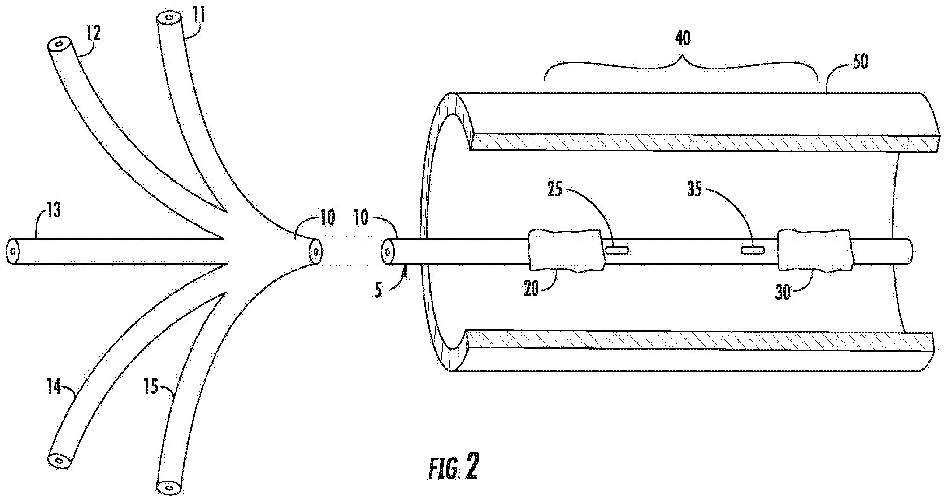

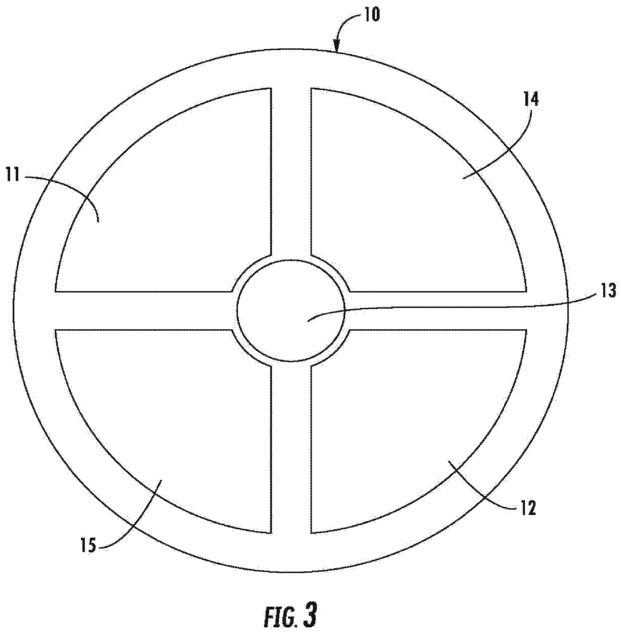

FIG. 2, for instance, illustrates a device for closing off a vein including the lumens, according to the invention. As shown in FIG. 2, for example, the lumen assembly 10 divides into individual lumens outside of the body vessel 50. The individual lumens include a distal balloon lumen 11, a proximal balloon lumen 12, an optional central lumen 13, an injection port lumen 14, and an aspiration port lumen 15. FIG. 3, for instance, illustrates a cross-section of the lumen assembly 10 in a device for closing off a vein, according to the invention. As shown in FIG. 3, the lumen assembly 10 channels each of the lumens 11-15 through a single tube such that the distal balloon lumen 11, the proximal balloon lumen 12, the injection port lumen 14, and the aspiration port lumen 15 are arranged around the optional central lumen 13 such that the two balloon lumens 11, 12 are positioned diagonally across from each other within the lumen assembly 10. The two port lumens 14, 15 are similarly positioned diagonally across from each other within the lumen assembly 10.

According to certain embodiments, for example, the central lumen 13 is configured to introduce the guide wire 5 into the body vessel 50. Prior to the guide wire 5 moving through the central lumen 13, the body vessel 50 may be prepared. For example, an incision in a patient's skin may be made, and the body vessel 50 may be opened at the location of the incision. The guide wire 5 may then be inserted into the opening in the body vessel 50 and threaded through the body vessel 50 under guidance of a visualization device (e.g., ultrasound 95), as described in more detail below. When the guide wire 5 reaches the appropriate treatment point within the body vessel 50, the central lumen 13 may be threaded over the guide wire 5 and into the body vessel 50. The central lumen 13 (and similarly the entire device) may be pushed along the length of the guide wire 5 until the distal balloon 30 is in a suitable location in the body vessel 50 as indicated by the visualization device.

The central lumen 13 is optional because a guide wire may be unnecessary. Guide wires are only necessary and required when the vessel poses a difficult pathway for insertion and positioning of the device due to the tortuosity of the vessel itself. In cases where the shape and path of the vessel requires use of a guide wire for insertion and position, the central lumen 13 becomes useful in allowing operation of the device.

According to the invention, the aspiration port 35 and the aspiration port lumen 15 is configured to remove at least one of blood, bodily fluid, a chemical agent, or any combination thereof from the treatment chamber 40. The injection port 25 and the injection port lumen 14 are configured to deliver a chemical agent to the treatment chamber 40.

Moreover, the proximal balloon 20 and the distal balloon 30 may be inflated through the proximal balloon lumen 12 and the distal balloon lumen 11 respectively. The balloons 20, 30 may be inflated using air or any other suitable fluid as understood by one of ordinary skill in the art. In this regard, the inflation of the balloons 20, 30 may secure the catheter in place and isolate the treatment chamber 40.

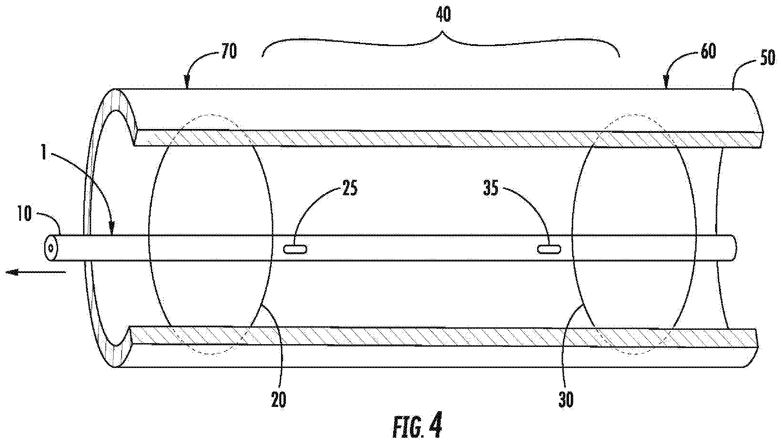

FIG. 4, for example, illustrates a partial view of a device 1 for closing off a vein in a deployed form according to the invention. As shown in FIG. 4, the balloons 20, 30 are inflated to define the treatment chamber 40. To define the treatment chamber 40, the proximal balloon 20 and the distal balloon 30 are configured to be inflated with a fluid. When inflated, the interior sides of the distal balloon 30 and the proximal balloon 20 (i.e. the sides facing internally towards each other) define the outer limits (e.g., starting point 60 and ending point 70) of the treatment chamber 40. For example, the proximal and distal balloons 20, 30 may be sized so that, when inflated, the outer ends of the balloons 20, 30 contact the interior surface of the body vessel 50 and form a seal, preventing fluids from entering or leaving the treatment chamber 40. According to certain embodiments, each of the distal balloon 30 and the proximal balloon 20 are spherical. However, the balloons 20, 30 may be any shape suitable for use in the device 1 as understood by one of ordinary skill in the art.

In some embodiments, for instance, the distal balloon 30 may comprise an inflated distal balloon diameter, the proximal balloon 20 may comprise an inflated proximal balloon diameter, and each of the inflated distal balloon diameter and the inflated proximal balloon diameter may be from about 5 mm to about 20 mm. In further embodiments, for example, each of the inflated distal balloon diameter and the inflated proximal balloon diameter may be from about 7 mm to about 15 mm. In other embodiments, for instance, each of the inflated distal balloon diameter and the inflated proximal balloon diameter may be from about 8 mm to about 12 mm. As such, in certain embodiments, each of the inflated distal balloon diameter and the inflated proximal balloon diameter may be from at least about any of the following: 5, 6, 7, and 8 mm and/or at most about 20, 19, 18, 17, 16, 15, 14, 13, and 12 mm (e.g., about 6-18 mm, about 5-14 mm, etc.).

Optionally, the device 1 may be provided with an inner balloon. The inner balloon may be sized so that it fills some of the volume inside the treatment chamber 40, but does not abut against the walls of the vessel 50. When the inner balloon is inflated, the inner balloon occupies some of the area inside the treatment chamber 40 while still allowing fluids to reach the walls of the body vessel 50 in the treatment chamber 40. Thus, the inner balloon effectively reduces the available volume inside the treatment chamber 40, which allows the treatment chamber 40 to be filled using a smaller amount of chemical agent. The length of the inner balloon may be dependent upon the length of the treatment chamber 40. In some embodiments, a small amount of space may be left between the inflated distal/proximal balloons 20, 30 and the inflated inner balloon. According to one embodiment, this space may be 1-10 mm in length, but the present invention is not limited to any particular sizes.

In another example, a method for secluding a body vessel is provided, wherein the method is not part of the claimed invention. In accordance with certain embodiments, the method may include removing blood from a treatment chamber 40 in the body vessel 50 via an aspiration port 35, delivering a chemical agent to the treatment chamber 40 via an injection port 25, maintaining the chemical agent in the treatment chamber 40 for a predetermined period of time to seclude the body vessel 50 within the treatment chamber 40, and removing the chemical agent from the treatment chamber 40 via the aspiration port 35. The aspiration port 35 may be operably coupled to an aspiration port lumen 15 of a vessel seclusion device (e.g., catheter), and the injection port 25 may be operably coupled to an injection port lumen 14 of the vessel seclusion device.

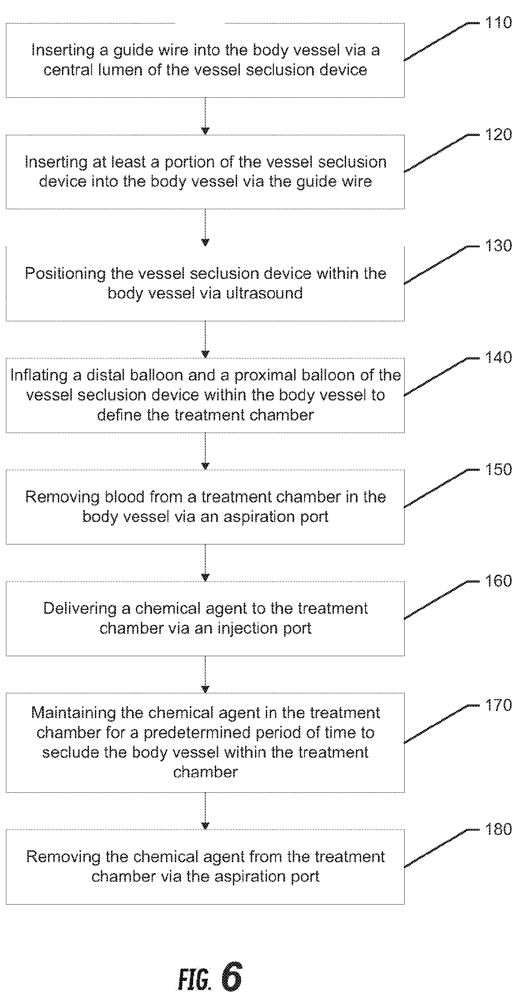

FIG. 6, for example, illustrates a block diagram of a method of secluding a body vessel 50 according to an example embodiment, not part of the claimed invention. As shown in FIG. 6, for instance, the method includes the initial steps of inserting a guide wire 5 into the body vessel 50 via a central lumen 13 of the vessel seclusion device at operation 110, inserting at least a portion of the vessel seclusion device into the body vessel 50 via the guide wire 5 at operation 120, positioning the vessel seclusion device within the body vessel 50 via ultrasound 95 at operation 130, and inflating a distal balloon 30 and a proximal balloon 20 of the vessel seclusion device within the body vessel 50 to define the treatment chamber 40 at operation 140. The method continues with the primary treatment steps of removing blood from the treatment chamber 40 in the body vessel 50 via an aspiration port 35 at operation 150, delivering a chemical agent to the treatment chamber 40 via an injection port 25 at operation 160, maintaining the chemical agent in the treatment chamber 40 for a predetermined period of time to seclude the body vessel 50 within the treatment chamber 40 at operation 170, and removing the chemical agent from the treatment chamber 40 via the aspiration port 35 at operation 180.

In accordance with certain embodiments, for instance, the chemical agent may be any agent known to chemically damage the body vessel 50 into which the catheter has been introduced, thereby causing the body vessel 50 to narrow or close. According to the invention, the chemical agent is a sclerosing agent typically used in sclerotherapy is and may include, but may not be limited to, polidocanol, sotradecol, hypertonic saline, or any other chemical agent suitable for damaging the vessel in the context of the sclerosing effect as understood by one of ordinary skill in the art. Alternatively or in addition, for instance, the chemical agent may be an agent known to elicit a biological response from the body vessel 50 into which the catheter has been introduced. In such embodiments, for example, the agent may be selected so as to induce the biological reaction substantially immediately after the catheter is withdrawn from the treatment chamber 40 (i.e. the body vessel 50 closes or narrows around the catheter as the catheter is withdrawn from the body vessel 50). In this regard, the body vessel 50 may be caused to immediately close following application of the chemical agent (as compared to traditional sclerotherapies, in which the vessel may take several days, or even several weeks, to close following application of the sclerosing agent).

According to certain embodiments, for example, after evacuating the treatment chamber 40 of all blood and/or other bodily fluids via the aspiration port lumen 15 and the aspiration port 35, a chemical agent may be introduced into the treatment chamber 40 via the injection port lumen 14 and injection port 25 and then maintained in the treatment chamber 40 for a predetermined amount of time due to the inflated balloons 20, 30. **In** certain embodiments in which an inner balloon is employed, the inner balloon may be inflated to reduce the available fluid volume inside the treatment chamber. **In** some embodiments, for instance, maintaining the chemical agent in the treatment chamber for the predetermined period of time may comprise maintaining the chemical agent in the treatment chamber for up to one minute (i.e. from about 1 second to about 60 seconds). As such, in certain embodiments, for instance, the chemical agent may be maintained in the treatment chamber for a time from at least about any of the following: 1, 5, 10, 20, 30, 40, 50, and 60 seconds and/or at most 60 seconds (e.g., about 5-60 seconds, about 30-60 seconds, etc.). **In** this regard, the chemical agent remains in contact with walls of the body vessel 50 in the treatment chamber 40 for a sufficient amount of time to seclude the body vessel 50 without being diluted or washed away by the flow of fluid in the body vessel 50 shortly after introduction of the chemical agent.

After the predetermined period of time, the chemical agent may be removed from the body vessel 50 via the aspiration port 35 and the aspiration port lumen 15. In some embodiments in which an inner balloon is deployed, the inner balloon may first be deflated, and any remaining chemical agent may be evacuated via the aspiration port 35. If the treatment chamber 40 fully encompassed the area to be secluded, the balloons 20, 30 may be deflated and the catheter may be withdrawn from the body vessel 50 through the original incision. The original incision in the body vessel 50 and/or skin may then be closed (e.g., via sutures). In this regard, the body vessel 50 may be secluded between two points (e.g., the starting point 60 and the ending point 70 in FIG. 4).

Following treatment, a patient will typically be capable of walking immediately and can return home after the procedure (i.e. the patient does not need to remain in a hospital overnight). The body vessel 50 may be secluded immediately, as opposed to conventional sclerotherapy, which may require additional time following treatment and/or multiple treatments in order to seclude the body vessel 50, and the body vessel 50 may be absorbed into surrounding tissue over a period of several months. The patient may be scheduled for a follow-up visit to verify that the body vessel 50 has been properly secluded and absorbed. If a problem is noted at the follow-up visit, for instance, the patient may undergo another round of treatment using the methods and devices disclosed herein or may be treated using a different method. In this regard, the methods disclosed herein may be used in combination with other conventional treatments.

In yet another example, another method for secluding a body vessel is provided, this other method also not being part of the claimed invention. In accordance with certain embodiments, the method may include selecting a seclusion length of the body vessel such that the seclusion length has a starting point and an ending point, dividing the seclusion length into at least two treatment chambers, secluding the first treatment chamber with a vessel seclusion device (e.g., catheter), moving the vessel seclusion device to the second treatment chamber, and secluding the second treatment chamber. The first treatment chamber may be defined by the starting point and a first intermediate point, and the second treatment chamber may be defined by the first intermediate point and the ending point. Secluding each of the first treatment chamber and the second treatment chamber may comprise removing blood from the treatment chamber in the body vessel via an aspiration port, delivering a chemical agent to the treatment chamber via an injection port, maintaining the chemical agent in the treatment chamber for a predetermined period of time to seclude the body vessel within the treatment chamber, and removing the chemical agent from the treatment chamber via the aspiration port as previously discussed herein. The aspiration port may be operably coupled to an aspiration port lumen of a vessel seclusion device, and the injection port may be operably coupled to an injection port lumen of the vessel seclusion device.

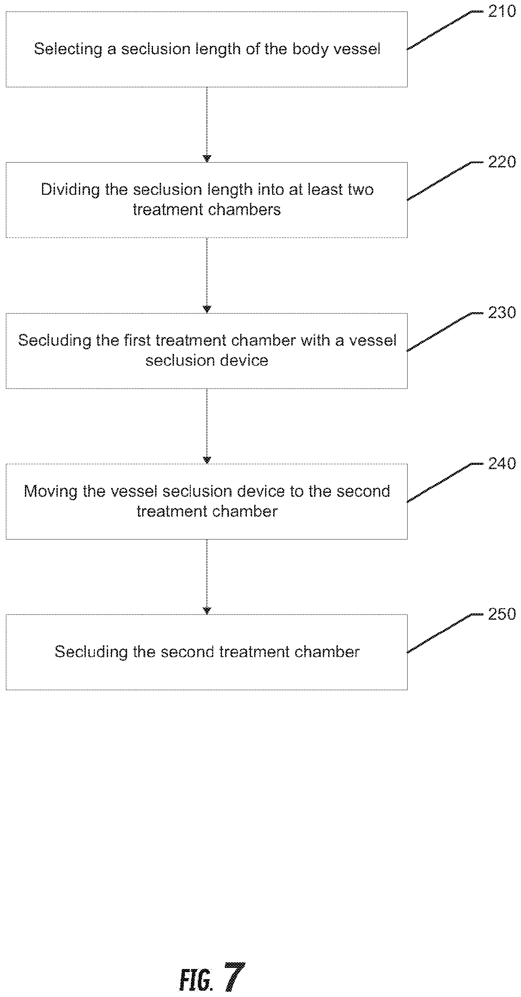

FIG. 7, for example, illustrates a block diagram of a method of secluding a body vessel according to an example embodiment, not part of the claimed invention. As shown in FIG. 7, for instance, the method includes selecting a seclusion length of the body vessel at operation 210, dividing the seclusion length into at least two treatment chambers at operation 220, secluding the first treatment chamber with a vessel seclusion device at operation 230, moving the vessel seclusion device to the second treatment chamber at operation 240, and secluding the second treatment chamber at operation 250. In this regard, if the seclusion length 100 extends beyond one treatment chamber 40, the catheter may be partially withdrawn in order to reposition the treatment chamber 40 at a new location along the seclusion length 100.

According to certain embodiments, for instance, the seclusion length 100 may be from about 3 cm to about 100 cm. In other embodiments, for example, the seclusion length 100 may be from about 7 cm to about 90 cm. In further embodiments, for instance, the seclusion length 100 may be from about 15 cm to about 80 cm. In some embodiments, for example, the seclusion length 100 may be from about 60 cm to about 70 cm. As such, in certain embodiments, the seclusion length 100 may be from at least about any of the following: 3, 5, 7, 10, 15, 20, 30, 40, 50, and 60 cm and/or at most about 100, 95, 90, 85, 80, 75, and 70 cm (e.g., about 5-70 cm, about 50-60 cm, etc.).

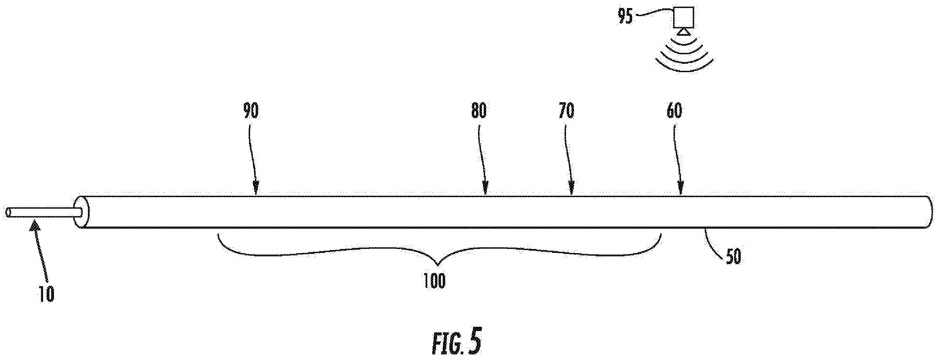

In accordance with certain embodiments, for example, the seclusion length may comprise at least three treatment chambers. In such embodiments, for instance, the first treatment chamber may be defined by the starting point and the first intermediate point, the second treatment chamber may be defined by the first intermediate point and a second intermediate point, and a third treatment chamber may be defined by the second intermediate point and the ending point. FIG. 5, for instance, illustrates a body vessel with an identified area to be secluded according to an example embodiment. As shown in FIG. 5, for example, the seclusion length 100 is divided into three treatment chambers, with the first treatment chamber to be located between the starting point 60 and the first intermediate point 70, the second treatment chamber to be located between the first intermediate point 70 and the second intermediate point 80, and the third treatment chamber to be located between the second intermediate point 80 and the ending point 90.

According to certain embodiments, moving the vessel seclusion device comprises positioning the vessel seclusion device within the body vessel 50 via ultrasound 95. Ultrasound 95 may be used to position the vessel seclusion device because it is not invasive and does not require special equipment to be deployed on the catheter or guide wire 5.

In this regard, the catheter may initially be positioned such that the treatment chamber 40 lies between the starting point 60 and the first intermediate point 70. Following application of the chemical agent between these points 60, 70, the catheter may be repositioned so that the distal balloon 30 is positioned at the first intermediate point 70 and the proximal balloon 20 is positioned at the second intermediate point 80. The method may be repeated until the full seclusion length 100 has been treated such that the proximal balloon 20 is positioned at the ending point 90.

In addition, it should be understood that aspects of the various embodiments may be interchanged in whole or in part. Furthermore, those of ordinary skill in the art will appreciate that the foregoing description is by way of example only, and it is not intended to limit the invention as further described in such appended claims.

## Claims

1. A device (1) for closing off a vein (50), comprising:
a distal balloon (30);
a proximal balloon (20);
an aspiration port (35) positioned adjacent to the distal balloon (30);
an injection port (25) positioned adjacent to the proximal balloon (20); and
a lumen assembly (10), at least a portion of which is configured to guide the device into position inside the vein (50), said lumen assembly (10) comprising a distal balloon lumen (11) operably coupled to the distal balloon (30), a proximal balloon lumen (12) operably coupled to the proximal balloon (20), an aspiration port lumen (15) operably coupled to the aspiration port (35), and an injection port lumen (14) operably coupled to the injection port (25),
wherein the lumen assembly (10) channels each of the lumens (11, 12, 14, 15) through a single tube such that the distal and proximal balloon lumens (11, 12), and the aspiration and injection port lumens are arranged around a centre of a cross-section of the lumen assembly, such that the distal and proximal balloon lumens are positioned diagonally across from each other within the lumen assembly (10) and the aspiration and injection port lumens (14, 15) are positioned diagonally across from each other within the lumen assembly (10);
wherein the distal balloon (30) and the proximal balloon (20) are separately inflatable, wherein inflating the distal balloon (30) and the proximal balloon (20) comprises separately inserting a fluid into the distal balloon (30) via the distal balloon lumen (11) and inserting a fluid into the proximal balloon (20) via the proximal balloon lumen (12),
wherein, when the device is positioned inside the vein, the distal balloon (30) and the proximal balloon (20) are configured to engage the vein (50) to define a treatment chamber (40) therebetween inside of the vein when each of the distal balloon (30) and the proximal balloon (20) are inflated, and the aspiration port (35) and the injection port (25) are positioned within the treatment chamber (40) on the lumen assembly (10),
wherein the device is not provided with an inner balloon between the distal balloon (30) and the proximal balloon (20),
wherein the aspiration port (35) and the aspiration port lumen (15) are configured to remove at least one of blood, bodily fluid, a sclerosing agent, or any combination thereof from the treatment chamber (40),
wherein the injection port (25) and the injection port lumen (14) are configured to deliver a sclerosing agent to the treatment chamber (40), and
wherein, when the device is positioned inside the vein and when each of the distal balloon (30) and the proximal balloon (20) are inflated, the distal balloon (30) and the proximal balloon (20) are configured to maintain a sclerosing agent in the treatment chamber (40) for a sufficient amount of time to seclude the vein (50) within the treatment chamber (40).

2. The device (1) of claim 1, further comprising a central lumen (13) configured to introduce a guide wire (5) into the vein (50).

3. The device (1) of claim 2, wherein the distal balloon lumen (11), the proximal balloon lumen (12), the injection port lumen (14), and the aspiration port lumen (15) are arranged around the central lumen (13).

4. The device (1) of any preceding claim, wherein each of the aspiration port (35) and the injection port (25) comprise a port orifice and a one-way valve at the port orifice.

5. The device (1) of any preceding claim, wherein each of the distal balloon (30) and the proximal balloon (20) are spherical.

6. The device (1) of any preceding claim, wherein the distal balloon (30) comprises an inflated distal balloon diameter, the proximal balloon (20) comprises an inflated proximal balloon diameter, and each of the distal balloon diameter and the proximal balloon diameter is from about 5 mm to about 20 mm.

7. The device (1) of any preceding claim, wherein the treatment chamber (40) comprises a length from about 3 cm to about 15 cm.

## Patentansprüche

1. Vorrichtung (1) zum Verschluss einer Vene (50), umfassend:
einen distalen Ballon (30);
einen proximalen Ballon (20);
einen Aspirationsport (35), der neben dem distalen Ballon (30) positioniert ist;
einen Injektionsport (25), der neben dem proximalen Ballon (20) positioniert ist; und
eine Lumenanordnung (10), von der mindestens ein Abschnitt dazu ausgelegt ist, die Vorrichtung im Innern der Vene (50) in Position zu führen, wobei die Lumenanordnung (10) ein distales Ballonlumen (11) umfasst, das funktional mit dem distalen Ballon (30) gekoppelt ist, ein proximales Ballonlumen (12), das funktional mit dem proximalen Ballon (20) gekoppelt ist, ein Aspirationsportlumen (15), das funktional mit dem Aspirationsport (35) gekoppelt ist, und ein Injektionsportlumen (14), das funktional mit dem Injektionsport (25) gekoppelt ist,
wobei die Lumenanordnung (10) jedes der Lumen (11, 12, 14, 15) durch eine einzelne Röhre kanalisiert, so dass das distale und das proximale Ballonlumen (11, 12) und das Aspirationsportlumen und das Injektionsportlumen um ein Zentrum eines Querschnitts der Lumenanordnung angeordnet sind, so dass das distale und das proximale Ballonlumen in der Lumenanordnung (10) einander diagonal gegenüber positioniert sind und das Aspirationsportlumen und das Injektionsportlumen (14, 15) in der Lumenanordnung (10) einander diagonal gegenüber positioniert sind;
wobei der distale Ballon (30) und der proximale Ballon (20) separat aufblasbar sind, wobei das Aufblasen des distalen Ballons (30) und des proximalen Ballons (20) das separate Einführen eines Fluids in den distalen Ballon (30) über das distale Ballonlumen (11) und das Einführen eines Fluids in den proximalen Ballon (20) über das proximale Ballonlumen (12) umfasst,
wobei, wenn die Vorrichtung im Innern der Vene positioniert ist, der distale Ballon (30) und der proximale Ballon (20) dazu ausgelegt sind, die Vene (50) in Eingriff zu nehmen, um eine Behandlungskammer (40) dazwischen im Innern der Vene zu definieren, wenn jeder des distalen Ballons (30) und des proximalen Ballons (20) aufgeblasen ist, und der Aspirationsport (35) und der Injektionsport (25) in der Behandlungskammer (40) an der Lumenanordnung (10) positioniert sind,
wobei die Vorrichtung nicht mit einem inneren Ballon zwischen dem distalen Ballon (30) und dem proximalen Ballon (20) bereitgestellt ist,
wobei der Aspirationsport (35) und das Aspirationsportlumen (15) dazu ausgelegt sind, mindestens eins von Blut, Körperflüssigkeit, einem Verödungsmittel oder einer Kombination davon aus der Behandlungskammer (40) zu entfernen,
wobei der Injektionsport (25) und das Injektionsportlumen (14) dazu ausgelegt sind, ein Verödungsmittel an die Behandlungskammer (40) zu liefern, und
wobei, wenn die Vorrichtung im Innern der Vene positioniert ist und wenn jeder des distalen Ballons (30) und des proximalen Ballons (20) aufgeblasen ist, der distale Ballon (30) und der proximale Ballon (20) dazu ausgelegt sind, ein Verödungsmittel für eine ausreichende Zeitdauer in der Behandlungskammer (40) zu halten, um die Vene (50) in der Behandlungskammer (40) zu veröden.

2. Vorrichtung (1) nach Anspruch 1, ferner umfassend ein zentrales Lumen (13), das dazu ausgelegt ist, einen Führungsdraht (5) in die Vene (50) einzuführen.

3. Vorrichtung (1) nach Anspruch 2, wobei das distale Ballonlumen (11), das proximale Ballonlumen (12), das Injektionsportlumen (14) und das Aspirationsportlumen (15) um das zentrale Lumen (13) angeordnet sind.

4. Vorrichtung (1) nach einem vorhergehenden Anspruch, wobei jeder des Aspirationsports (35) und des Injektionsports (25) eine Portöffnung und ein Einwegventil an der Portöffnung umfasst.

5. Vorrichtung (1) nach einem vorhergehenden Anspruch, wobei jeder des distalen Ballons (30) und des proximalen Ballons (20) kugelförmig ist.

6. Vorrichtung (1) nach einem vorhergehenden Anspruch, wobei der distale Ballon (30) einen Durchmesser des aufgeblasenen distalen Ballons umfasst, der proximale Ballon (20) einen Durchmesser des aufgeblasenen proximalen Ballons umfasst, und jeder des distalen Ballondurchmessers und des proximalen Ballondurchmessers von etwa 5 mm bis etwa 20 mm beträgt.

7. Vorrichtung (1) nach einem vorhergehenden Anspruch, wobei die Behandlungskammer (40) eine Länge von etwa 3 cm bis etwa 15 cm umfasst.

## Revendications

1. Dispositif (1) d'obturation d'une veine (50), comprenant :
un ballonnet distal (30) ;
un ballonnet proximal (20) ;
un orifice d'aspiration (35) positionné adjacent au ballonnet distal (30) ;
un orifice d'injection (25) positionné adjacent au ballonnet proximal (20) ; et
un ensemble lumière (10), dont au moins une partie est conçue pour guider le dispositif en position à l'intérieur de la veine (50), ledit ensemble lumière (10) comprenant une lumière de ballonnet distal (11) accouplée de manière fonctionnelle au ballonnet distal (30), une lumière de ballonnet proximal (12) accouplée de manière fonctionnelle au ballonnet proximal (20), une lumière d'orifice d'aspiration (15) accouplée de manière fonctionnelle à l'orifice d'aspiration (35), et une lumière d'orifice d'injection (14) accouplée de manière fonctionnelle à l'orifice d'injection (25),
dans lequel l'ensemble lumière (10) canalise chacune des lumières (11, 12, 14, 15) à travers un tube unique de telle sorte que les lumières de ballonnets distal et proximal (11, 12), et les lumières d'orifice d'aspiration et d'injection soient agencées autour du centre d'une section transversale de l'ensemble lumière, de telle sorte que les lumières de ballonnet distal et proximal soient positionnées en diagonale l'une par rapport à l'autre à l'intérieur de l'ensemble lumière (10) et que les lumières d'orifices d'aspiration et d'injection (14, 15) soient positionnées en diagonale l'une par rapport à l'autre à l'intérieur de l'ensemble lumière (10) ;
dans lequel le ballonnet distal (30) et le ballonnet proximal (20) sont gonflables séparément, dans lequel le gonflage du ballonnet distal (30) et du ballonnet proximal (20) comprend, séparément, l'insertion d'un fluide dans le ballonnet distal (30) par le biais de la lumière de ballonnet distal (11) et l'insertion d'un fluide dans le ballonnet proximal (20) par le biais de la lumière de ballonnet proximal (12),
dans lequel, lorsque le dispositif est positionné à l'intérieur de la veine, le ballonnet distal (30) et le ballonnet proximal (20) sont conçus pour entrer en prise avec la veine (50) pour définir une chambre de traitement (40) entre eux à l'intérieur de la veine lorsque chacun du ballonnet distal (30) et du ballonnet proximal (20) est gonflé, et l'orifice d'aspiration (35) et l'orifice d'injection (25) sont positionnés à l'intérieur de la chambre de traitement (40) sur l'ensemble lumière (10),
dans lequel le dispositif n'est pas pourvu d'un ballonnet interne entre le ballonnet distal (30) et le ballonnet proximal (20),
dans lequel l'orifice d'aspiration (35) et la lumière d'orifice d'aspiration (15) sont conçus pour retirer de la chambre de traitement (40) au moins parmi du sang, un fluide corporel, un agent sclérosant ou une combinaison quelconque de ceux-ci,
dans lequel l'orifice d'injection (25) et la lumière d'orifice d'injection (14) sont conçus pour administrer un agent sclérosant à la chambre de traitement (40), et
dans lequel, lorsque le dispositif est positionné à l'intérieur de la veine et que chacun du ballonnet distal (30) et du ballonnet proximal (20) est gonflé, le ballonnet distal (30) et le ballonnet proximal (20) sont conçus pour maintenir un agent sclérosant dans la chambre de traitement (40) pendant une durée suffisante pour isoler la veine (50) à l'intérieur de la chambre de traitement (40).

2. Dispositif (1) selon la revendication 1, comprenant en outre une lumière centrale (13) conçue pour introduire un fil guide (5) dans la veine (50).

3. Dispositif (1) selon la revendication 2, dans lequel la lumière de ballonnet distal (11), la lumière de ballonnet proximal (12), la lumière d'orifice d'injection (14) et la lumière d'orifice d'aspiration (15) sont agencées autour de la lumière centrale (13).

4. Dispositif (1) selon une quelconque revendication précédente, dans lequel chacun de l'orifice d'aspiration (35) et de l'orifice d'injection (25) comprend un trou d'orifice et une soupape unidirectionnelle au niveau du trou d'orifice.

5. Dispositif (1) selon une quelconque revendication précédente, dans lequel chacun du ballonnet distal (30) et du ballonnet proximal (20) est sphérique.

6. Dispositif (1) selon une quelconque revendication précédente, dans lequel le ballonnet distal (30) comprend un diamètre de ballonnet distal gonflé, le ballonnet proximal (20) comprend un diamètre de ballonnet proximal gonflé, et chacun du diamètre de ballonnet distal et du diamètre de ballonnet proximal est d'environ 5 mm à environ 20 mm.

7. Dispositif (1) selon une quelconque revendication précédente, dans lequel la chambre de traitement (40) comprend une longueur d'environ 3 cm à environ 15 cm.
